# EUROPEAN PATENT SPECIFICATION

(11) **EP 2 332 611 B1**
(45) Date of publication and mention of the grant of the patent: **17.06.2020**
(21) Application number: 09015333.9
(22) Date of filing: 10.12.2009
(51) Int. Cl.: A61M 39/28, F16K 7/06

(54) **Irreversible flow control clamp for flexible tubes**
Irreversibel schliessbare Durchflussregelungsklemme für flexible Schläuche
Pince régulatrice de débit irréversible pour des tuyaux flexibles

(43) Date of publication of application: 15.06.2011
(73) Proprietor: Fenwal, Inc., Lake Zurich, IL 60047 (US)
(72) Inventor: Mathias, Jean-Marie, Lilois 1428 Belgium (BE)
(74) Representative: Geary, Stephen

(56) References cited:
- EP-A1- 0 206 997
- EP-A2- 1 000 633
- WO-A1-03/063945
- WO-A1-2007/112500
- WO-A2-2007/133291

## Description

This invention relates to an irreversible flow control clamp and a fluid processing set including an irreversible flow control clamp.

A disposable plastic container and tubing set or fluid circuit is typically used for collecting blood from a donor. The disposable blood collection set includes a venipuncture needle for insertion into the arm of the donor. The needle is attached to one end of a flexible plastic tube which provides a flow path for the blood. The other end of the plastic tube is attached to one or more plastic bags or containers for collecting the withdrawn blood.

The blood collection set may also include a sampling sub-unit. The sampling sub-unit allows for collection of a sample of blood, which sample can be used for testing of the blood. Preferably, the sample is obtained prior to the "main" collection of blood. Collecting the sample prior to the main collection reduces the risk that bacteria residing on the donor's skin where the needle is inserted (i.e., in particular, the small section of detached skin commonly referred to as the "skin plug") will enter the collection container and contaminate the blood collected for transfusion. Thus, it is preferred that the blood sample, which may include the skin plug, be diverted from the main collection container.

An example of a blood collection set with such a "pre-donation" sampling sub-unit is described in U.S. Patent Nos. 6,387,086 and 6,520,948, which are incorporated by reference herein. The collection sets described therein include a needle and a length of tubing, one end of which is attached to the needle and the other end of which is attached to one or more collection containers. The tubing set also includes an additional line which is branched from the main line at a Y-connection site in the tubing set. The branched line is attached to a sampling pouch for collecting a smaller volume of blood from which samples may be obtained.

The sampling sub-unit may further include a pre-attached holder for receiving blood sample vials or tubes. The holder is connected to the outlet port of the sampling pouch and includes a needle in the holder interior. When the blood sample vial is inserted into the holder, the needle pierces the rubber cap (septum) of the vial and blood from the sampling pouch is drawn into the vial. The blood-filled vial is removed from the holder and the procedure may be repeated with as many vials as required.

The blood collection set described above also includes flow control clamps for controlling the flow of biological fluid (e.g., blood) through the set and to the sampling pouch and/or collection container. Flow control clamps commonly used are the Roberts-type clamps, which are well known in the art. The Roberts-type clamps are placed on the tubing line leading to the blood collection container and on the tubing line leading to the sampling pouch. A Roberts-type clamp is typically made from a strip of plastic. The ends of the strip are curved toward each other to provide two "legs" which are adapted to engage each other in a snap-fit, spring relation. The body of the clamp includes a pair of apertures through which the tubing passes.

The clamp further includes a pair of projections or tube contacting members which compress the tubing when the body of the clamp is depressed, thereby restricting flow through the tube. Clamps of this type are generally described in U.S. Pat. Nos. 3,942,228, 6,089,527 and 6,113,062, all of which are incorporated herein by reference.

The clamps described in these patents also typically include means for locking the legs together and release means which, when actuated by the technician, disengages the locked legs of the flow control clamp. Disengagement of the ends decompresses the tube and permits fluid flow through the flow path. Thus, by selectively opening and closing the different flow paths (by depressing or releasing the clamps), the technician can control the flow of blood from the donor, diverting the blood to the desired container or sampling pouch, as necessary.

In certain instances, however, it may not be desirable to reopen a closed flow path. This may particularly be the case when drawing blood samples from the sampling pouch into sample vials. For example, when the sampling pouch is filled with the required or desired volume of blood (for sampling), the line from the donor to the pouch is typically closed by the Roberts-type clamp, and remains closed as the sampling vials are filled. After clamping the sampling line closed, technicians are further instructed to more permanently seal the line using metal clips or by heat-sealing the line in ways that will be known to those in the field. Sealing the sampling line substantially ensures that airborne bacteria or other contaminants that may enter the set through the sampling sub-unit do not contaminate the remainder of the set, including the needle and tube leading to the collection container. In short, the clips or heat seal provide a sterile barrier to the remainder of the set.

A wide range of clamps are known. For example WO2007/133291 describes a clamp having two plates for receiving tubing and a locking mechanism with two hooks for engaging with one of the plates to close the clamp. The configuration provides a significant area for the user or some other object to grab onto and attempt to pull the legs apart and open the clamp which runs a risk that the clamp may not close irreversibly.

WO2007/112500 describes a clamp having opposed jaws joined by a hinge and adapted to receive and clamp a tube which passes between the jaws. The locking mechanism extends from a central projection.

EP-A-206997 describes a device for severing a tube. The device includes projections 16 and 17 however, only one of the projections is engaged at a time and does not provide for multiple locking mechanisms to be simultaneously engaged in an irreversible manner.

Unfortunately, on occasion, prior to permanently sealing the sampling line, some technicians have been known to unclamp the sampling line in order to draw additional blood into the pouch so that additional sample vials may be filled. However, by doing so, the system becomes "open" and contaminants introduced from the outside environment may enter the system. Current flow control clamps are readily and easily openable, and do not provide a disincentive to drawing additional blood into the sampling pouch. If the clamp could not be so readily released from the closed position, the technician would not have the option of reopening the line but would, hopefully, proceed with permanently sealing the line to form the sterile barrier. Accordingly, it would be desirable to provide a clamp that discourages and substantially prevents the reopening of the sampling or other blood tube line.

### SUMMARY

The invention is defined by the subject-matter of claim 1; particular embodiments are defined by the subject-matter of the dependent claims.

In one aspect, the present invention provides an irreversible flow control clamp as defined in claim 1. flow The invention is directed to a clamp for controlling flow through a fluid circuit. The clamp includes a flexible body having a central portion and first and second legs extending from the central portion. The legs are disposed relative to each other in a generally facing relationship and the body includes a pair of apertures for receiving flexible tubing therethrough. One of the apertures is disposed in the central portion and the other of the apertures is disposed in at least one the legs. The legs are movable from a first open spaced apart position to a second closed irreversibly locked position. First and second locking mechanisms are associated with the legs and the first 3 locking mechanism is positioned farther from the central portion than the secondary locking mechanism.

In another aspect, the present disclosure not part of the invention is directed to a fluid processing set. The set includes a container adapted for receiving biological fluid from a donor, a donor access device and a tubing segment which defines a flow path that is in flow communication with the container and the access device. The set further includes a flow control clamp associated with the tubing segment. The clamp includes a flexible body having a central portion and first and second legs extending from the central portion. The legs are disposed relative to each other in a generally facing relationship and the body includes a pair of apertures for receiving the tubing segment therethrough. One of the apertures is disposed in the central portion and the other of the apertures is disposed in at least one the legs. The legs are movable from a first open spaced apart position to a second closed irreversibly locked position. First and second locking mechanisms are associated with the legs and the first locking mechanism is positioned farther from the central portion than the secondary locking mechanism.

Suitably at least one and preferably both the first and second locking mechanisms comprise interengaging locking structures on the legs. Desirably the interengaging locking structures of the first locking mechanism are provided at terminal ends of each of the legs. Preferably the interengaging locking structure of the first locking mechanism comprises a pair of spaced apart walls defining a slot on the first leg, and the second leg including an extension, whereby movement of the legs from the spaced apart position to the closed position introduces the extension into the slot.

The secondary locking mechanism advantageously provides further means for ensuring the clamp is closed irreversibly, in addition to the first locking mechanism. The secondary locking mechanism may also advantageously provide one or more additional benefits including further enhancing the structural rigidity of the clamp so reducing the risk or preventing distortion of the clamp during use and acting as a guide for threading the tubing passing through the pair of apertures.

Preferably at least one, and desirably both, locking mechanisms comprise interengaging locking structures on the legs. Suitably, the interengaging locking structures of the first locking mechanism are provided at terminal ends of each of the legs and optionally comprises a pair of spaced apart walls defining a slot on the first leg, and the second leg including an extension, whereby movement of the legs from a spaced apart position to a closed position introduces the extension into the slot. Where present, the interengaging locking structures of the second locking mechanism may comprise one of the legs having a ledge and the other leg having a catch for engaging the ledge in the said closed position. In a further embodiment, the interengaging locking structures of the second locking mechanism may comprise a first engaging member extending from one of the legs and a second engaging member extending from the other leg. Preferably, the first engaging member comprises a catch disposed thereon and said second engaging member comprises a ledge for engaging the catch in the closed position. In a preferred embodiment, the first engaging member includes at least one arm or a pair of arms extending from one of the legs. Optionally one or both of the first and second engaging members further comprises a contacting member for compressing a tube when the legs are in the closed position.

Preferably, at least one and desirably both the apertures in the clamp are completely closed.

The clamp includes at least one tube contacting member carried by one of the legs for clamping the tubing segment when the legs are in the irreversibly locked position and desirably, both legs comprise a tube contacting member for clamping the tubing segment in the closed position.

### BRIEF DESCRIPTION OF THE FIGURES

FIG. 1 is a perspective view of a blood tubing set including a flow control clamp as disclosed herein;
FIG. 2 is a perspective view of a flow control clamp as disclosed herein;
FIG. 3 is a plan view of the flow control clamp of FIG. 2 taken along lines 3-3 of FIG. 2;
FIG. 4 is a plan view of the flow control clamp of FIG. 2 taken along lines 4-4 of FIG. 2;
FIG. 5 is a plan view of the flow control clamp of FIG. 2 along lines 5-5;
FIG. 6 is a plan view of the flow control clamp of FIG. 2 taken along lines 6-6;
FIG. 7 is a cross-sectional, side view of the flow control clamp of FIG. 2 in the open position;
FIG. 8 is a cross-sectional, side view of the flow control clamp of FIG. 2 in the irreversibly locked position.
FIG. 9 is a side view of an alternative embodiment of the flow control clamp as disclosed herein;
FIG. 10 is a side view of another alternative embodiment of the flow control clamp as disclosed herein;
FIG. 11 is a side view of another alternative embodiment of the flow control clamp as disclosed herein;
FIG. 12 is a side view of still another alternative embodiment of the flow control clamp as disclosed herein;
FIG. 13 is a perspective view of a flow control clamp including first and second locking mechanisms as disclosed herein;
FIG. 14 is a perspective view of the flow control clamp of FIG. 13 with a flexible tube extending therethrough;
FIG. 15 is a cross-sectional, side view of the flow control clamp of FIG. 13 in the open position;
FIG. 16 is a cross-sectional, side view of the flow control clamp of FIG. 15 in the irreversibly locked position; and
FIG. 17 is a cross-sectional, end view of the flow control clamp of FIG. 16 taken along line 16-16.

### DETAILED DESCRIPTION

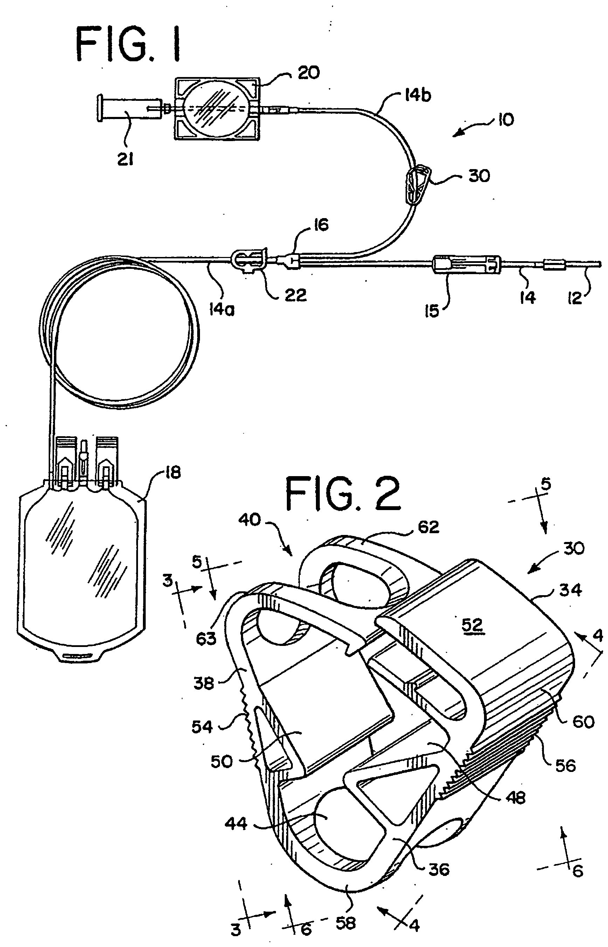

With reference to the drawings, FIG. 1 depicts a blood collection set 10 of known type that is used in the collection of blood from a donor. Tubing set 10 includes a venipuncture needle 12 and a length of tubing 14. Tubing 14 branches at for example, Y-connector 16 into tubing segment 14a and tubing segment 14b. Tubing segment 14a provides a flowpath to a collection container 18 and tubing segment 14b provides a flow path to a second container, such as, a sampling pouch 20. The sampling pouch may also include a holder 21 for receiving a blood sampling vial or tube. (Also shown is a needle protector 15 for storing the needle after use.)

In the blood tubing sets of the type shown in FIG. 1, tubing segments 14a and 14b are passed through flow control clamps 22 and 30 which may be a standard Roberts-type clamp and an irreversibly closable flow control clamp embodying the present invention. As used herein, the terms "irreversibly" closed, closable or locked means that the flow control clamp, once in the closed position, is not readily releasable from the closed position in the normal and intended mode of operation. An "irreversibly" closed, or closable or locked flow control clamp can only be released from the closed and locked position by extraordinary and unintended manipulation of the clamp, including breakage of the clamp. A flow control clamp that is "irreversibly" closed, closeable or locked restricts flow through the flow path that extends through the clamp and does not allow for flow to be re-established without extraordinary or unintended manipulation. A flow control clamp that is "irreversibly" closed or closable includes no release members such as, but not limited to, the release members described in U.S. Pat. Nos. 3,942,228, 6,089,527, 6,113,062.

Thus, for example, whereas flow control clamp 22 (e.g., a standard Roberts-type clamp) can be selectively opened and closed, as desired, clamp 30, which is the subject of the present disclosure, once closed, remains irreversibly closed, as defined above.

Turning now to FIG. 2, flow control clamp 30 of the present disclosure includes a generally curved, bent or otherwise non-linear body 34 (described in more detail below). Preferably, flow control clamp 30 may be made of any flexible, moldable, plastic material that can be steam sterilized. In addition, flow control clamp 30 and body 34 should be made of a material that is strong, yet sufficiently flexible so that the clamp can be flexed and squeezed by the technician without breaking. Examples of suitable materials are many of the known plastic materials typically used in the medical field, including, but not limited to polyoxymethylene and polypropylene. Most preferred of the above-identified materials is polyoxymethylene, such as the polyoxymethylene known as Hostaform.TM. available from Ticona of Frankfurt, Germany. In addition, other materials that can be sterilized by other forms of sterilization, such as gamma sterilization, may also be used. One such material is a terpolymer of acrylonitrile, butadiene and styrene (ABS).

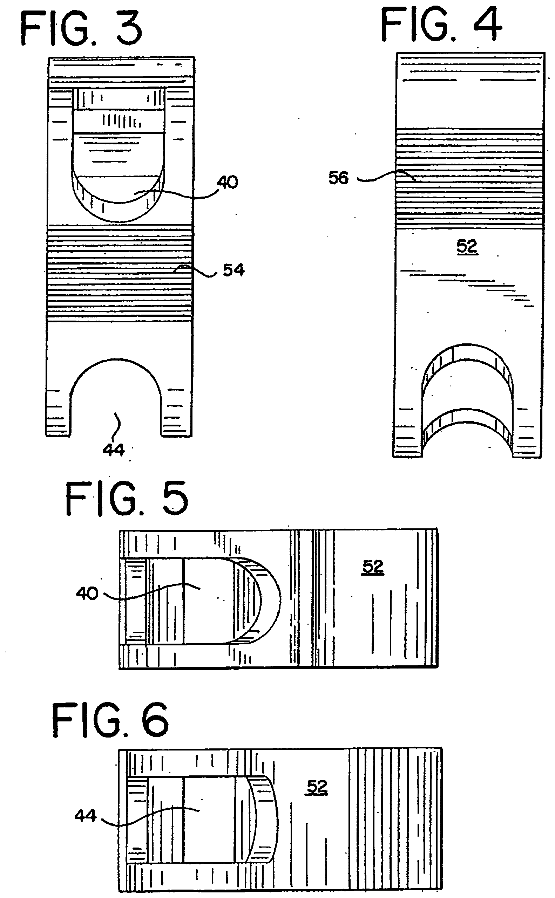

As further shown in FIG. 2, flow control clamp 30 includes body 34 that has a central portion 35 and two legs 36 and 38 extending therefrom. Legs 36 and 38 are disposed in a generally facing relationship relative to each other. Legs 36 and 38 are initially in a spaced apart position as shown in FIG. 4, and are movable from the first open spaced apart position toward each other to ultimately a second irreversibly closed or locked position as shown in FIG. 5.

Clamp body 34 further includes apertures 40 and 44 for receiving a length of plastic tubing therethrough. In the illustrated embodiment, aperture 44 is defined within the central portion 35 of body 30 and aperture 40 is defined in leg 38; however, it will be appreciated that the aperture may be defined in leg 40 or by a combination of the legs. As shown in FIG. 2, apertures 40 and 44 are preferably circular or oval-like and are entirely contained within body 34 of flow control clamp 30. Thus, when a length of tubing 43 is inserted through apertures 40 and 44, the rim defining the aperture completely encloses and surrounds the tubing at the point where the tubing extends through the apertures. Also, it may be preferable that apertures 40 and 44 be generally aligned with each other and not substantially offset relative to one another. This provides, among other things, for easier threading of the length of tubing 43. Alternatively, apertures need not completely surround the tube, but instead may include lateral slots, as described, for example, in U.S. Pat. No. 6,113,062. The lateral slots extend to the outer edge of the body so that during insertion of the tube, the clamp can be laterally slipped on the tube (as opposed to threading).

Flow control clamp 30 includes one or more tube contacting members 48 and 50. Tube contacting members 48 and 50 are carried by legs 36 and 38, respectively. As shown in FIG. 2, tube contacting members 48 and 50 may be teeth-like projections which compress the tubing when the clamp is closed. Alternatively, tube contacting members 48 and 50 may simply be blunt, oppositely facing surfaces. Although two oppositely facing contacting members are shown in FIG. 2 and are preferred, it will be understood that flow control clamp 30 of the present disclosure may include only one tube contacting member carried by one of the legs 36 or 38.

The outer surface 52 of flow control clamp 30 is generally flat and smooth. However, outer surface 52 may include portions that are roughened or textured to provide some friction when contacted by the fingertips of the technician during operation. As shown in FIGS. 3 - 5, outer surface 52 of body 34 includes ribbed portions 54 and 56, which provide such a frictional surface for contacting by the fingers of the technician during closure and compression of flow control clamp 30.

As best shown in FIGS. 4 and 5, flow control clamp 30 is substantially free of any sharp corners or edges. For example, bends 58, 60 and 62 in body 34 are not abrupt, sharp corners, but are gradually curved and rounded. In addition, edges 63 of body 34 are preferably rounded or beveled. A flow control clamp 30 that is substantially free of sharp corners and edges and is more rounded or beveled, like the flow control clamp shown in FIGS. 4 and 5, ensures that the flow control clamp will not puncture the packaging of blood processing sets, thereby maintaining sterility of the packaged set.

As discussed generally above, where the blood processing set includes a sampling pouch of the type shown in FIG. 1, it may be desirable to provide a flow control clamp 30 that provides an additional level of sterility maintenance by discouraging and/or perhaps even preventing the technician from reopening a previously closed clamp. This way, the sterility of the remainder of the blood processing set will be maintained and will not be compromised by bacteria from the outside environment, or the collected sample in sample pouch 20 will not be diluted by, for example, anticoagulant in the fluid set.

Accordingly, flow control clamp 30 includes a locking mechanism, and more particularly, legs 36 and 38 include surfaces that are disposed for irreversibly securing together legs 36 and 38 when the legs of flow control clamp 30 are moved from a first spaced-apart position to the second closed position, as shown in FIGS. 4 and 5 respectively.

Many different ways for securing legs 36 and 38 together to prevent easy reopening by the technician are contemplated by the present disclosure. For example, flow control clamp 30 may include means on the leg surfaces for interlocking legs 36 and 38. Specifically, as shown in FIGS. 4 and 5, leg 36 of flow control clamp 30 may include a pair of spaced-apart walls 66 and 68 that depend or otherwise extend from leg 36 at a distal end of the clamp. Spaced-apart walls 66 and 68 define a slot 70 for receiving an extension 72 carried by leg 38. One of the spaced-apart walls 66 or 68 may further include a lip 74 that projects into slot 70, while the extension 72 includes a hook 76 that projects toward the wall 66.

As the legs 36 and 38 are moved from the first open spaced-apart position, as generally depicted in FIG. 4, to the second closed irreversibly locked position, tube contacting members 48 and 50 compress the tube 43, thereby restricting flow therethrough. As flow control clamp 30 is compressed further, extension 72 is outwardly displaced by wall 66 and enters slot 70. As hook 76 contacts lip 74, extension 72 is again outwardly displaced until it clears lip 74. Once clear of lip 74, extension 72 snaps forward toward wall 66, and hook 76 engages lip 74 to effectively close and lock flow control clamp 30.

Wall 68 prevents release or further outward displacement of extension 72 from the closed position, thereby resulting in an "irreversibly closed" flow control clamp 30. As shown in FIG. 5, wall 68 is a thicker and a less flexible wall than, for example, wall 66, thereby providing more strength to wall 68 and making it even more difficult to release extension 72. As will be appreciated by those in the field, flow control clamp 30 does not include any tabs or other means for readily releasing extension 72. Thus, flow control clamp 30 remains in the second closed position. In addition, clamp 30 lacks any easy-to-grasp surface or member that can be used to pry open the clamp when it is in the second closed position. For example, clamps that have tabs or outwardly extending members or surfaces may be more susceptible to re-opening by being grasped by a user or accidentally caught on an object and used as a lever arm to pry open the clamp. In contrast, the interengaging member of the locking mechanism (e.g., walls 66, 68 and extension 72) as described above are contoured relative to each other and are in sufficiently close association so as to be devoid of any easy-to-grasp surface or member to allow grasping or other manipulation that could result in relatively easy re-opening of the clamp. Accordingly, the compact and contained body of clamp 30 with the locking mechanism protected at the distal end assists in preventing inadvertent or even intentional opening of the clamp once in the second closed position.

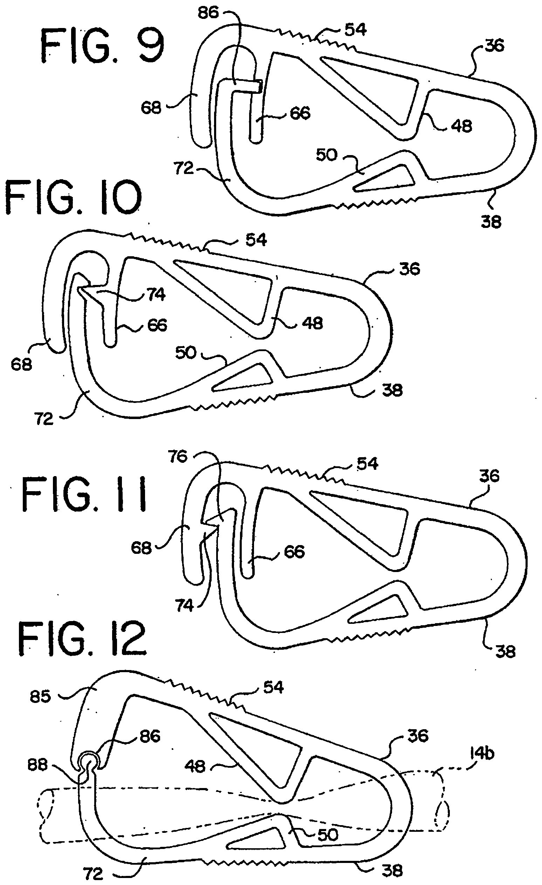

FIGS. 9-12 include additional means for securing legs 36 and 38 in an "irreversibly closed" position. For example, as shown in FIG. 9, extension 72 may include a peg, and wall 66 includes a mating notch 84 for receiving peg 86 of extension 72 at a distal end of clamp 30. Extension 72 enters slot 70 in the manner generally described above, until peg 86 snaps into notch 84. Wall 68 again prevents outward movement of extension 72, thereby retaining flow control clamp 30 in an "irreversibly closed" position.

FIG. 10 is an alternative embodiment where extension 72 includes a notch for receiving lip 74 of wall 66. In FIG. 11, lip 74 is located on wall 68 and is engaged by hook 76, as previously described.

FIG. 12 shows yet another alternative embodiment of the flow control clamp 30 of the present disclosure wherein leg 36 includes a single wall 85 projecting toward the interior (i.e., toward leg 38) of the flow control clamp 30. Leg 38 includes an extension, likewise projecting toward the interior (i.e., and toward leg 36) of the flow control clamp 30. In order to secure legs 36 and 38 in the "irreversibly closed" position, one of the legs may include a socket 86, and the other of the legs may include a ball 88 for insertion into socket 86 at a distal end of clamp 30. This way, as in the previous embodiments, flow control clamp 30 may be closed or locked in the "irreversibly closed" position.

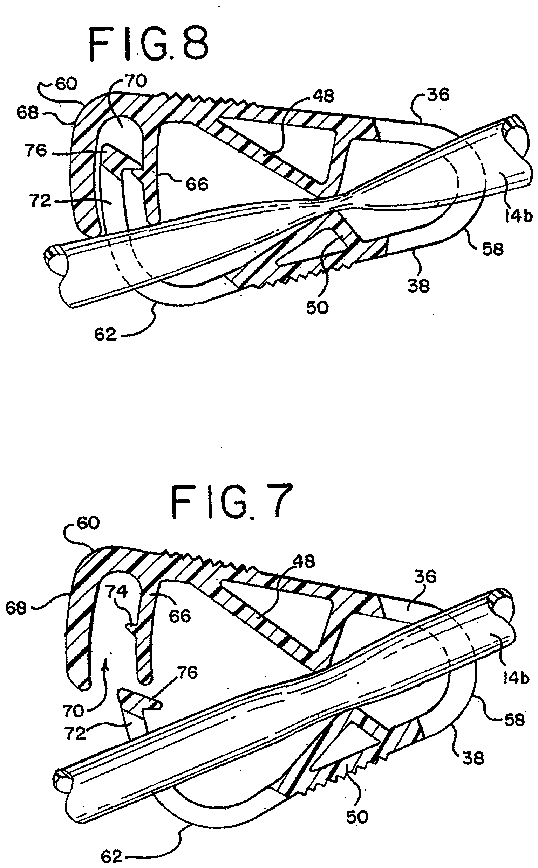

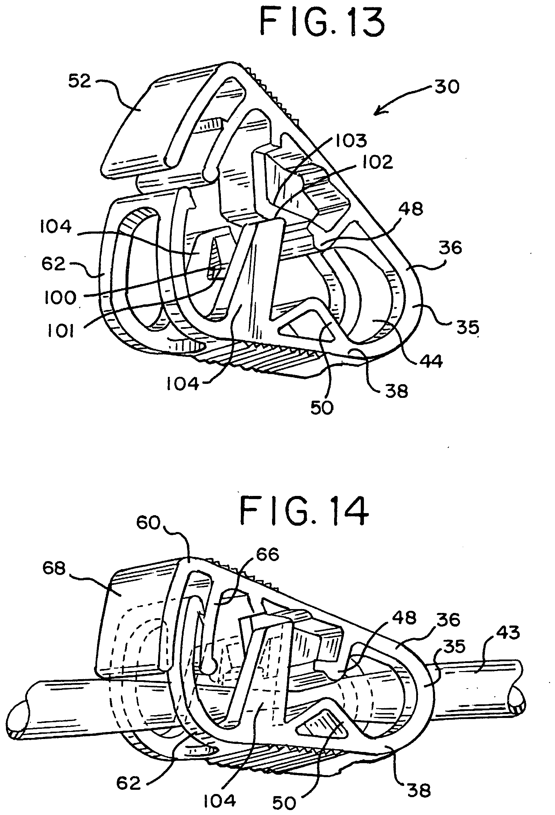

FIGS 13-17 illustrate a further alternative embodiment of flow control clamp 30 which includes among other things, a second locking mechanism that can be used in combination with the first locking mechanism as described above. The flow control clamp of FIGS 13-17 is similar to the flow control clamp of FIGS 1-12 and similar or identical elements will be referred to with identical reference numerals. The flow control clamp of Figures 13 to 17 may be employed with a fluid processing set, for example a blood collection set as shown in Figure 1.

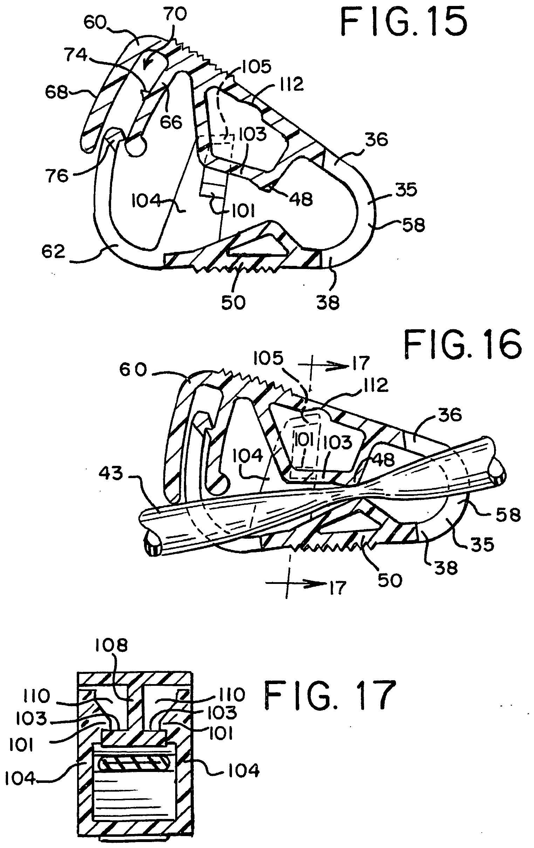

As in the clamps described in Figures 2 to 12 above, a clamp illustrated in FIGS 13 - 17 includes a central portion 35 and pair of inwardly movable and facing legs 36 and 38. Similarly, clamp 30 of FIGS 13 - 17 includes one or more tube contacting members 48 and 50. The illustrated embodiment also preferably includes lateral and preferably parallel arms 104 that extend from the sides of leg 38 as best seen in FIGS. 13 and 17. Lateral arms 104 may act as a guide for threading the tubing 43 through apertures 40 and 44 of the clamp and help maintain (e.g., center) the tubing within the clamp body and more specifically, between contacting members 48 and 50 (see FIG. 14). In addition, arms 104 may prevent distortion of the clamp when in the closed position. It will be appreciated that arms 104 need not extend from the outermost surface of leg 38; however, the arms should be spaced sufficiently apart such that tube 43 can freely pass between the arms without arms 104 substantially pressing against tubing 43 so as to, for example, restrict fluid flow through the tube. Leg 36 may further include a groove or cutout 112 for receiving or otherwise accommodating distal end tip105 of one or both lateral arms 104. As will be discussed in more detail below, arms 104 may also carry one or more locking mechanisms. In an alternative embodiment, clamps provided in accordance with the present disclosure may include a "first" locking mechanism and lateral arms 104 without a locking mechanism associated with the arms (or without a second locking mechanism whatsoever) although a clamp with first and second locking mechanisms as described below is preferred.

As noted above, clamp 30 of FIGS 13-17, includes a first locking mechanism as described above in connection with FIGS 2-12. The first locking mechanism shown in the embodiments of Figure 2-7, 8, 9, 10, 11 or 12 may be employed as the first locking mechanism in combination with a second locking mechanism, for example as illustrated in FIGS 13 -17.

The clamp 30 of Figures 13 to 17 may include walls 66 and 68 depending from one of legs 36 and 38 to define a slot 70. The first locking mechanism of clamp 30 may include the interengaging extension 72 and hook 76 arrangements of FIGS. 2, 7-8 or 11, or alternatively, the peg 86 and notch 84 arrangement of FIG. 9, the notch and lip arrangement of FIG. 10, the ball 88 and socket 86 arrangement of FIG. 12 or any other comparable mechanism for engaging one of legs 36 or 38 with the other of legs 36 or 38. As described in connection with the clamp of FIGS 2-12, this compact clamp arrangement assists in preventing inadvertent or even intentional opening of the clamp once in the second closed position.

In addition to such first locking mechanism, clamp 30 may include a second locking mechanism that is spaced from the first locking mechanism. For example, in one embodiment a second locking mechanism may be positioned closer to central portion 35 than the first locking mechanism. For example, whereas first-locking mechanism(s) are generally located at a distal end of clamp 30, second locking mechanism is preferably spaced from the first locking mechanism. More preferably, the second locking mechanism is proximally located relative to the first locking mechanism and distally positioned relative to central portion 35. Thus, in a preferred embodiment, second locking mechanism is positioned between the first locking mechanism and central portion 35.The second locking mechanism may be positioned on either side of the contacting members (e.g., distal or proximal to contacting members). In one embodiment, second locking mechanism may include interengaging structures that are disposed on or otherwise associated with legs 36 and 38 when the legs of flow control clamp 30 are moved from a first spaced-apart position to the second irreversibly closed position, as shown in FIGS. 13 and 14 respectively.

More specifically, in an embodiment, the second locking mechanism includes first engaging member 100 associated with leg 38 and a second engaging member 102 associated with leg 36. First engaging member 100 preferably includes catch 101 and second engaging member 102 preferably includes ledge 103. As best seen in FIG. 17, ledge 103 extends laterally from central support member 108 that depends from leg 36. As flow control clamp 30 is moved from the first open position to the second closed position, catch 101 is outwardly displaced until it clears ledge 103. Once clear of ledge 103, catch 101 engages the top of ledge 103 to assist (first locking mechanism described above) in locking flow control clamp 30 in its second irreversibly closed position. The interaction between catch 101 and ledge 103 is best illustrated in FIG. 17. It is appreciated that one skilled in the art would recognize other structures that may be used on the engaging members to lock the legs together in the second position, for example, a ball and socket.

As shown in FIGS. 13-17, catch 101 is more particularly positioned on (parallel) lateral arms 104 that extend from opposite sides of leg 38. In the illustrated embodiment, the arms are formed such that as clamp 30 is moved from the first open position to the second closed position, the end tip 105 of the arms fit into a recess 110 formed by ledge 103 and support 108 (see FIG. 17). This allows leg 36 to act as a stop and limit the travel of arms 104. Alternatively, it will be appreciated that only one arm and catch may be provided in the flow control clamp 30 of FIGS 13-17. It will be appreciated that the outer sides 120 of lateral arms 104 do not extend beyond the edges of legs 36 and 38, as best shown in FIG. 17. This, likewise provides clamp 30 with a compact and contained body that has no easy-to-grasp outwardly extending members or surfaces that would allow one to grasp and pry open clamp 20.

Ledge 103 on leg 36 may be integral with contacting member 48; however, it will also be appreciated that ledge 103 may be separate from contacting member 48. In addition, it will be understood that arms 104 may be integral with contacting member 50 and that positioning catch 101 and ledge 103 can be reversed such that ledge 103 is associated with leg 38 and catch 101 is associated with leg 36.

Regardless of the relative location of the interengaging elements of the second locking mechanism, a clamp having both first and second locking mechanisms spaced apart from each other and relatively spaced from central portion 35 provides more secure clamping. The dual locking mechanism of a clamp described above and as shown in FIGS. 13-17 would make it more difficult to re-open the clamp inadvertently or even intentionally. In other words, spacing the first and second locking mechanisms away from each other, i.e., provides engagement of legs 36 and 38 at two distinct locations. In addition, spacing the first and second locking mechanisms away from each other may allow for sequential engagement i.e., engagement of one of the locking mechanism followed by engagement of the other of the locking mechanism. The sequential engagement can be accompanied by audible "clicks" whereby a first audible "click" followed by a second audible "click" thereby providing assurance to the user that the clamp is locked in the "irreversibly closed" position. Finally, providing two separate locking mechanisms allows for a back-up locking mechanism in the event one of the two locking mechanisms fails.

It will be appreciated that the description set forth above has been offered for illustrative purposes only. Other embodiments and other modifications to the flow control clamp shown and described above will be readily apparent to one skilled in the art and may also be included within the scope of the present disclosure. The above description is not intended to limit the scope of the invention of this application, which is as defined in the claims below.

## Claims

1. A flow control clamp 30 comprising:
a flexible body comprising a central portion 35 and first and second legs 36, 38
extending from said central portion 35, said legs disposed relative to each other in a general facing relationship, leg 38 including first and second lateral arms 104 extending from respective sides thereof;
said body including a pair of apertures 40, 44 for receiving flexible tubing therethrough wherein one of said apertures 40, 44 is disposed in said central portion 35 and the other of said apertures 40, 44 is disposed in at least one of said legs 36, 38;
wherein said legs 36, 38 are movable from a first open spaced apart position to a second closed irreversibly locked position;
at least one tube contacting member 48. 50 carried by one of said legs 36, 38 for
compressing a tube 43 when said legs 36, 38 are in the closed position;
first and second locking mechanisms comprising interengaging locking structures associated with said legs 36, 38, wherein said interengaging locking structures are irreversibly engaged when said legs 36, 38 are in the closed position, said interengaging locking structures of said first locking mechanism comprising first and second interengaging arms (62, 66), and
each interengaging arm (62, 66) comprising an engaging member to irreversibly lock the first locking mechanism when in the closed position;
said interengaging locking structures of said second locking mechanism comprises a first engaging member 100 on said arms 104 associated with leg 38 and a second engaging member 102 associated with leg 36;
wherein said first locking mechanism is positioned farther from said central portion than said second locking mechanism.

2. The flow control clamp of claim 1 wherein said interengaging locking structures of said first locking mechanism are provided at terminal ends of each of said legs 36, 38.

3. The flow control clamp of claim 1 or 2 wherein said interengaging locking structures of said first locking mechanism further comprises a wall (68), the wall (68) and one of the first and second interengaging arms (66) defining a slot 70 on said first leg 36, and said second leg 38 including an extension 76, whereby movement of said legs 36, 38 from said spaced apart position to said closed position introduces said extension 76 into said slot 70.

4. The flow control clamp of any one of claims 1 to 3 wherein said interengaging locking structures of said second locking mechanism comprises one of said legs 36 having a ledge 103 and said other leg 38 having a catch 101 for engaging a ledge in said closed position.

5. The flow control clamp of any one of claims 2 or 3 wherein said interengaging locking structures of said second locking mechanism comprises a first engaging member 100 extending from one of said legs and a second engaging member 102 extending from said other leg.

6. The flow control clamp of any one of claims 1 to 5 wherein said apertures 40, 44 are completely enclosed.

7. The flow control clamp of claim 3 wherein one of said spaced-apart walls 66, 68 is more rigid than said other of said spaced-apart walls.

8. The flow control clamp of any one of claims 1 to 7 further comprising a second tube contacting member carried by said other of said legs for compressing said tube between said contacting members when said legs 36, 38 are in the closed position.

9. The flow control clamp of claim 8 wherein one of said engaging members 100, 102 comprises said contacting member for compressing a tube when said legs are in the closed position.

## Patentansprüche

1. Durchflussregelungsklemme 30, umfassend:
einen flexiblen Körper, der einen zentralen Abschnitt 35 und einen ersten und
einen zweiten Schenkel 36, 38 umfasst, die sich von dem zentralen Abschnitt 35 erstrecken, wobei die Schenkel relativ zueinander in einer im Allgemeinen zugewandten Beziehung angeordnet sind, wobei der Schenkel 38 einen ersten und einen zweiten lateralen Arm, 104 umfasst, die sich von jeweiligen Seiten davon erstrecken;
wobei der Körper ein Paar Öffnungen 40, 44 zum Aufnehmen einer flexiblen Verschlauchung durch diese aufweist, wobei eine der Öffnungen 40, 44 in dem zentralen Abschnitt 35 angeordnet ist und die andere der Öffnungen 40, 44 in zumindest einem der Schenkel 36, 38 angeordnet ist;
wobei die Schenkel 36, 38 von einer ersten offenen beabstandeten Position zu einer zweiten geschlossenen irreversibel verriegelten Position beweglich sind;
zumindest ein Schlauchkontaktierungsglied 48, 50, das durch einen der Schenkel 36, 38 getragen ist, um ein Rohr 43 zusammenzudrücken, wenn sich die Schenkel 36, 38 in der geschlossenen Position befinden;
einen ersten und einen zweiten Verriegelungsmechanismus, umfassend ineinandergreifende Verriegelungsstrukturen, die den Schenkeln 36, 38 zugeordnet bzw. mit diesen verbunden sind, wobei die ineinandergreifenden Verriegelungsstrukturen irreversibel in Eingriff sind, wenn sich die Schenkel 36, 38 in der geschlossenen Position befinden, wobei die ineinandergreifenden Verriegelungsstrukturen des ersten Verriegelungsmechanismus einen ersten und einen zweiten ineinandergreifenden Arm (62, 66) umfassen und jeder ineinandergreifende Arm (62, 66) ein Eingriffsglied umfasst, um den ersten Verriegelungsmechanismus in der geschlossenen Position irreversibel zu verriegeln;
wobei die ineinandergreifenden Verriegelungsstrukturen des zweiten Verriegelungsmechanismus ein erstes Eingriffsglied 100 an den Armen 104, die dem Schenkel 38 zugeordnet bzw. mit diesem verbunden sind, und ein zweites Eingriffsglied 102 umfassen, das dem Schenkel 36 zugeordnet bzw. mit diesem verbunden ist;
wobei der erste Verriegelungsmechanismus weiter von dem zentralen Abschnitt entfernt positioniert ist als der zweite Verriegelungsmechanismus.

2. Durchflussregelungsklemme nach Anspruch 1, wobei die ineinandergreifenden Verriegelungsstrukturen des ersten Verriegelungsmechanismus an Anschlussenden jedes der Schenkel 36, 38 bereitgestellt sind.

3. Durchflussregelungsklemme nach Anspruch 1 oder 2, wobei die ineinandergreifenden Verriegelungsstrukturen des ersten Verriegelungsmechanismus ferner eine Wand (68) umfassen, wobei die Wand (68) und einer der ersten und zweiten ineinandergreifenden Arme (66) einen Schlitz 70 an dem ersten Schenkel 36 definieren, wobei der zweite Schenkel 38 eine Verlängerung 76 enthält, wodurch eine Bewegung der Schenkel 36, 38 von der beabstandeten Position zu der geschlossenen Position die Verlängerung 76 in den Schlitz 70 einbringt.

4. Durchflussregelungsklemme nach einem der Ansprüche 1 bis 3, wobei die ineinandergreifenden Verriegelungsstrukturen des zweiten Verriegelungsmechanismus einen der Schenkel 36 mit einer Leiste 103 und den anderen Schenkel 38 mit einem Schnapper bzw. Riegel 101 zum Eingreifen in eine Leiste in der geschlossenen Position umfassen.

5. Durchflussregelungsklemme nach einem der Ansprüche 2 oder 3, wobei die ineinandergreifenden Verriegelungsstrukturen des zweiten Verriegelungsmechanismus ein erstes Eingriffsglied 100, das sich von einem der Schenkel erstreckt, und ein zweites Eingriffsglied 102 umfassen, das sich von dem anderen Schenkel erstreckt.

6. Durchflussregelungsklemme nach einem der Ansprüche 1 bis 5, wobei die Öffnungen 40, 44 vollständig umschlossen sind.

7. Durchflussregelungsklemme nach Anspruch 3, wobei eine der beabstandeten Wände 66, 68 steifer ist als die andere der beabstandeten Wände.

8. Durchflussregelungsklemme nach einem der Ansprüche 1 bis 7, ferner umfassend ein zweites Schlauchkontaktierungsglied, das durch den anderen der Schenkel getragen ist, um den Schlauch zwischen den Kontaktgliedern zusammenzudrücken, wenn sich die Schenkel 36, 38 in der geschlossenen Position befinden.

9. Durchflussregelungsklemme nach Anspruch 8, wobei eines der Eingriffsglieder 100, 102 das Kontaktierungsglied zum Zusammendrücken eines Schlauchs umfasst, wenn sich die Schenkel in der geschlossenen Position befinden.

## Revendications

1. Pince de commande de débit (30) comprenant :
un corps flexible comprenant une partie centrale (35) et des première et seconde pattes (36, 38) s'étendant à partir de ladite partie centrale (35), lesdites pattes étant disposées l'une par rapport à l'autre suivant une relation globalement en regard, la patte (38) comportant des premier et second bras latéraux (104) s'étendant à partir de ses côtés respectifs ;
ledit corps comportant une paire d'ouvertures (40, 44) destinées à recevoir une tuyauterie flexible à travers, dans laquelle l'une desdites ouvertures (40, 44) est disposée sur ladite partie centrale (35) et l'autre desdites ouvertures (40, 44) est disposée sur au moins l'une desdites pattes (36, 38) ;
dans laquelle lesdites pattes (36, 38) peuvent être déplacées à partir d'une première position espacée, ouverte, vers une seconde position fermée, verrouillée de manière irréversible ;
au moins un tube entrant en contact avec un élément (48, 50) supporté par l'une desdites pattes (36, 38) afin de compresser un tube (43) lorsque lesdites pattes (36, 38) sont dans la position fermée ;
des premier et second mécanismes de verrouillage comprenant des structures de verrouillage coopérantes associées auxdites pattes (36, 38), dans laquelle lesdites structures de verrouillage coopérantes sont couplées de manière irréversible lorsque lesdites pattes (36, 38) sont dans la position fermée, lesdites structures de verrouillage coopérantes dudit premier mécanisme de verrouillage comprenant des premier et second bras coopérants (62, 66), et chaque bras coopérant (62, 66) comprenant un élément de couplage destiné à verrouiller de manière irréversible le premier mécanisme de verrouillage lorsqu'il est dans la position fermée ;
lesdites structures de verrouillage coopérantes dudit second mécanisme de verrouillage comprennent un premier élément de couplage (100) sur lesdits bras (104) associé à la patte (38) et un second élément de couplage(102) associé à la patte (36) ;
dans laquelle ledit premier mécanisme de verrouillage est positionné plus loin de ladite partie centrale que ledit second mécanisme de verrouillage.

2. Pince de commande de débit selon la revendication 1, dans laquelle lesdites structures de verrouillage coopérantes dudit premier mécanisme de verrouillage sont agencées au niveau des extrémités terminales de chacune desdites pattes (36, 38).

3. Pince de commande de débit selon la revendication 1 ou 2, dans laquelle lesdites structures de verrouillage coopérantes dudit premier mécanisme de verrouillage comprennent, en outre, une paroi (68), la paroi (68) et l'un des premier et second bras coopérants (66) définissant une fente (70) sur ladite première patte (36), et ladite seconde patte (38) comportant une saillie (76), de telle sorte que le mouvement desdites pattes (36, 38) à partir de ladite position espacée vers ladite position fermée introduit ladite saillie (76) dans ladite fente (70).

4. Pince de commande de débit selon l'une quelconque des revendications 1 à 3, dans laquelle lesdites structures de verrouillage coopérantes dudit second mécanisme de verrouillage comprennent l'une desdites pattes (36) comportant un rebord (103) et ladite autre patte (38) comportant un élément de saisie (101) destiné à se coupler à un rebord dans ladite position fermée.

5. Pince de commande de débit selon l'une quelconque des revendications 2 ou 3, dans laquelle lesdites structures de verrouillage coopérantes dudit second mécanisme de verrouillage comprennent un premier élément de couplage (100) s'étendant à partir de l'une desdites pattes et un second élément de couplage (102) s'étendant à partir de l'autre desdites pattes.

6. Pince de commande de débit selon l'une quelconque des revendications 1 à 5, dans laquelle lesdites ouvertures (40, 44) sont complètement fermées.

7. Pince de commande de débit selon la revendication 3 ,dans laquelle l'une desdites parois espacées (66, 68) est plus rigide que ladite autre desdites parois espacées.

8. Pince de commande de débit selon l'une quelconque des revendications 1 à 7, comprenant, en outre, un second tube entrant en contact avec l'élément supporté par ladite autre desdites pattes afin de compresser ledit tube entre lesdits éléments de contact lorsque lesdites pattes (36, 38) sont dans la position fermée.

9. Pince de commande de débit selon la revendication 8, dans laquelle l'un desdits éléments de couplage (100, 102) comprend ledit élément de contact destiné à compresser un tube lorsque lesdites pattes sont dans la position fermée.
